# EUROPÄISCHE PATENTSCHRIFT

(11) **EP 1 706 415 B1**
(45) Veröffentlichungstag und Bekanntmachung des Hinweises auf die Patenterteilung: **05.10.2011**
(21) Anmeldenummer: 05714898.3
(22) Anmeldetag: 24.01.2005
(51) Int. Cl.: C07J 51/00, A61K 31/575, A61P 39/04, C23F 11/167, A61K 9/127

(54) **LIPID- DERIVATISIERTE BISPHOSPHONSÄURE**
LIPID- DERIVATIZED BISPHOSPHONIC ACID
DERIVES LIPIDIQUES D'ACIDE BISPHOSPHONIQUE

(30) Priorität: 23.01.2004 DE 102004003781
(43) Veröffentlichungstag der Anmeldung: 04.10.2006
(73) Patentinhaber: MCS Micro Carrier Systems GmbH, 41460 Neuss (DE)
(72) Erfinder: GREB, Wolfgang, 40489 Düsseldorf (DE); SHYHSKOV, Oleg, 28329 Bremen (DE); RÖSCHENTHALER, Gerd-Volker, 28357 Bremen (DE); HENGST, Verena, 40215 Düsseldorf (DE)
(74) Vertreter: Christophersen, Ruth
(86) Internationale Anmeldenummer: PCT/DE2005/000095
(87) Internationale Veröffentlichungsnummer: WO 2005/070952

(56) Entgegenhaltungen:
- EP-A- 0 555 845
- WO-A-86/00902
- WO-A-97/39004
- WO-A-97/49711
- US-A- 4 942 036
- SZAJNMAN SERGIO H ET AL: "Bisphosphonates derived from fatty acids are potent inhibitors of Trypanosoma cruzi farnesyl pyrophosphate synthase." BIOORGANIC & MEDICINAL CHEMISTRY LETTERS, Bd. 13, Nr. 19, 6. Oktober 2003 (2003-10-06), Seiten 3231-3235, XP002323677 ISSN: 0960-894X
- HSU, M.-T. ET AL: "Inhibition of streptococcal growth, F-ATPase and pyrophosphatase by diphosphonates" ORAL MICROBIOLOGY AND IMMUNOLOGY , 10(1), 47-53 CODEN: OMIMEE; ISSN: 0902-0055, 1995, XP008045394
- HIROZAWA, STANLEY T.: "Use of electrochemical noise in the study of corrosion inhibition of aluminum by gem-diphosphonates" ANNALI DELL'UNIVERSITA DI FERRARA, SEZIONE 5: CHIMICA PURA ED APPLICATA, SUPPLEMENTO , 10(8TH EUROPEAN SYMPOSIUM ON CORROSION INHIBITORS, 1995, VOL. 1), 25-33 CODEN: AUFSAH; ISSN: 0365-785X, 1995, XP008045407
- DATABASE CA [Online] CHEMICAL ABSTRACTS SERVICE, COLUMBUS, OHIO, US; PALADINI, M.: "Inhibition of metal catalysis in oil oxidation" XP002323678 gefunden im STN Database accession no. 1991:447975 & RIVISTA ITALIANA DELLE SOSTANZE GRASSE , 66(6), 335-40 CODEN: RISGAD; ISSN: 0035-6808, 1989,
- DATABASE CA [Online] CHEMICAL ABSTRACTS SERVICE, COLUMBUS, OHIO, US; ONDA, AKIO ET AL: "Manufacture of long fibers from plants including treatment by alkaline substances and hydrogen peroxide" XP002323679 gefunden im STN Database accession no. 1996:620880 & JP 08 199420 A2 (JAPAN) 6. August 1996 (1996-08-06)

## Beschreibung

Die vorliegende Erfindung betrifft Cholesteryl-3-hydroxy-bisphosphonsäureverbindungen und deren löslichen Salze oder Hydrate und pharmakologisch wirksame Konjugate, ein Verfahren zu ihrer Herstellung sowie deren Verwendung zur Behandlung von Erkrankungen.

### Hintergrund

### Phosphonsäure-Derivate und ihre technische Anwendung

Phosphonsäuren sind organische Bestandteile, die eine oder mehrere C-PO(OH)₂-Gruppe(n) mit stabilen, kovalenten Kohlenstoff-Phosphor-Bindungen aufweisen. Phosphonate sind effektive Chelat-Komplexbildner für di- und trivalente Metallionen. Die meisten Phosphonate sind den Aminocarboxylaten, wie EDTA, NTA und DTPA, sehr ähnlich. Außerdem inhibieren sie auch sehr wirksam Kristallwachstum und Korrosion.

Aufgrund dieser Eigenschaften werden sie bei zahlreichen technischen und industriellen Anwendungen eingesetzt. Ein wichtiger Einsatz in der Industrie ist ihr Gebrauch in Kühlwasser, Entsalzungssystemen und auf Ölfeldern, um die Korrosion zu inhibieren. Sowohl in der Textilindustrie als auch in der Papier- und Zellstoff-Herstellung werden Phosphonate als Stabilisatoren für Bleichmittel verwendet, indem sie als Chelat-Komplexbildner fungieren, die das Peroxid inaktivieren können. Ein Beispiel aus der Umwelt für den Gebrauch von Phosphonaten ist das Glyphosat (N-Phosphonomethylglycin), ein nicht-selektives Herbizid, welches das Pflanzenwachstum durch die Hemmung einer biochemischen Kaskade kontrolliert.

Polyphosphate stellen Polymere (Kondensationsprodukte) von Orthophosphat-Resten dar, die durch energiereiche Phosphoanhydrid-Bindungen (Sauerstoff-Brücken) gebunden sind. Polyphosphat (PolyP) wird im menschlichen Körper synthetisiert und ist in fast allen Zellen vorzufinden. Der größte Anteil an PolyP ist in den knochenbildenden Osteoblasten anzutreffen. PolyP hat viele Funktionen, abhängig davon, welcher Körperabschnitt in Betracht gezogen wird. Es speichert energiereiches Phosphat, komplexierendes Calcium oder andere divalente Kationen, es fungiert als Gegenion für basische Aminosäuren oder als Regulator des intrazellulären Spiegels von Adenylatnucleotiden.

PolyP wird häufig in Zahnpasten verwendet, weil angenommen wird, dass es die Kariesbildung verhindert was zurückgeführt wird auf die Fähigkeit, Hydroxylapatit mineralisieren zu können und sowohl dessen Azidität als auch dessen Löslichkeit verringern zu können.

Die Gruppe der Bisphosphonate wird zur Behandlung von verschiedenen Knochenerkrankungen und Erkrankungen, die den Calcium-Metabolismus betreffen, eingesetzt.

Bisphosphonate sind Pyrophosphat-Analoga, bei denen die Sauerstoffbrücke durch ein Kohlenstoffatom mit variierenden Seitenketten ersetzt wird. Die P-C-P Gruppe ist gegenüber enzymatischer Hydrolyse resistent, aus diesem Grund werden Bisphosphonate nicht im Körper metabolisiert. Bisphosphonate können in drei Generationen eingeteilt werden. Sie unterscheiden sich in der Substitution des Wasserstoffes durch verschiedene Seitenketten an zwei möglichen Positionen im Molekül. Alkyl-Seitenketten (z.B. Etidronat) charakterisieren die erste Generation. Die zweite Generation der Bisphosphonate umfasst die Amino-Bisphosphonate mit einer terminalen Aminogruppe (z.B. Alendronat). Seitenketten, die Ringe aufweisen, sind typisch für die dritte Generation (z.B. Zolendronat).

In der WO 97/39004 werden organische Phosphonsäurederivate offenbart, die eine verbesserte Bioverfügbarkeit für Wirkstoffe und Nahrungsmittel zeigen sollen. Die beanspruchten Phosphorverbindungen weisen neben dem Phosphonsäurerest eine Alkylgruppe mit mehr als zehn Kohlenstoffatomen auf.

In der EP 0 555 845 werden weitere Bisphosphonsäurederivate offenbart, die neben den Phosphonsäureresten und den Phosphonsäureesterresten als weitere Gruppe eine Steroidverbindung aufweisen.

In der WO 97/49711 werden Carboxykonjugate von geminalen Bisphosphonsäuren mit Alkylierungsmitteln offenbart. Diese Derivate sollen eine Anti-Tumor-Aktivität und eine spezifische Aktivität gegenüber der Knochenresorption zeigen.

### Medizinische Anwendungen von Phosphonaten

In der Knochen-Szintigraphie werden Phosphonate als Diagnostika eingesetzt. Einige verschieden markierte Phosphonate, wie z.B. ^{99m}TC-markierte Phosphonate oder ¹⁸⁸Re-Komplexe werden als radioaktive Marker verwendet, um im Skelett das Vorhandensein, den Ort und das Ausmaß von Krankheiten, wie Osteomyelitis, Knochen-Neoplasien, Arthritis oder von Knocheninfarkten darzustellen.

Die wichtigste pharmakologische Wirkung von Bisphosphonaten ist die Hemmung der Knochenresorption. Sie haben wie das Pyrophosphat eine hohe Affinität zum Hydroxylapatit, dem Hauptbestandteil vom Knochen, und verhindern sowohl dessen Wachstum als auch dessen Auflösung. Ausserdem inaktivieren sie knochenabbauende Zellen, Osteoklasten genannt, indem sie ihre Apoptosis herbeiführen. Normalerweise arbeiten die Osteoklasten mit den knochenaufbauenden Zellen, den Osteoblasten, zusammen, um den bestehenden Knochen wieder aufzubauen. Sie visieren Knochenareale an, die eine hohe Osteoklastenaktivität aufweisen und sie tragen dazu bei, dass das normale Verhältnis zwischen Osteoblasten- und Osteoklasten-Aktivität wiederhergestellt wird.

Bisphosphonate kommen in der Therapie von Knochenkrankheiten zum Einsatz, zumeist bei Morbus Paget, Hypercalcämie, Osteoporose und Neoplasien.

Ein weiterer Vorteil dieser Gruppe ist, dass sie die Apoptose von Tumorzellen bewirken können. Deshalb spielen sie in der Krebstherapie eine große Rolle (z.B. bei Brustkrebs, bei Metastasen, bedingt durch Prostatakrebs, oder beim Multiplen Myelom).

Derivate, die aus acyclischen nucleosidischen Phosphonaten bestehen (z.B. Zidofovir oder Tenofovir) sind wirksam gegen eine große Vielfalt von DNA-Viren und Retroviren verursachte Erkrankungen. Acyclische nukleosidische Phosphonate (ANPs) sind Analoga, bei denen ein Phosphonat über eine aliphatische Kette durch eine Etherbindung an ein Purin oder ein Pyrimidin gebunden ist. Sobald diese Analoga in der Zelle phosphoryliert werden, konkurrieren sie mit den natürlich vorkommenden Nukleotiden bei der Nukleinsäure Synthese, folglich wird die Virusreplikation in den infizierten Zellen herabgesetzt oder verhindert.

Die antivirale Wirksamkeit von ANPs wird auch in der Tiermedizin ausgenutzt. Sie sind potente Inhibitoren des Feline Immunodeficiency Virus (FIV). FIV ähnelt dem HI-Virus in Bezug auf morphologische, physikalische und biochemische Eigenschaften.

### Zielgerichtete Applikation von Wirkstoffen / zielgerichtete Liganden

Aufgrund der außergewöhnlichen Affinität der Bisphosphonate zu Hydroxylapatit wurde auch ihre Eignung für die zielgerichtete Applikation von pharmakologisch wirksamen Substanzen am Knochen untersucht. Ein Beispiel dafür ist die Verbindung von dem Bisphosphonat, welches eine hohe Affinität zum Knochen aufweist, und Wachstumsfaktoren (z.B. Bovines Serum Albumin), die die Fähigkeit besitzen, das Knochenwachstum zu stimulieren. Radioisotope, antineoplastische Wirkstoffe und anti-inflammatorische Substanzen sind auch bereits an diese zielgerichteten Liganden gebunden worden

Der Ausdruck "zielgerichtete Wirkstoff-Applikation" umfasst Substanzen, die eine zeitkontrollierte Abgabe, eine organspezifische Applikation, Schutz, verlängerte in vivo Wirkung und eine Abnahme der Toxizität der Wirkstoffe ermöglichen. Viele Trägersysteme, wie z.B. Polymere, Nanopartikel, Mikrospheren, Mizellen, Protein-Trägersysteme, DNA-Komplexe, wie auch Liposomen, sind angewendet worden, um die Zirkulationszeit von verschiedenen Molekülen zu verlängern, um sie an die gewünschten Wirkorte zu bringen, und um sie vor dem Abbau im Plasma zu schützen. Liposomen wurden bisher als Wirkstoffträger sehr vielseitig eingesetzt. Sie weisen kolloide, vesikuläre Strukturen auf der Basis von (Phospho)-Lipid-Doppelmembranen auf. Wegen dieser strukturellen Eigenschaften können sie sowohl hydrophile als auch hydrophobe Moleküle einlagern. Außerdem sind Liposomen bioabbaubar und im Wesentlichen ungiftig, da sie aus natürlichen Biomolekülen bestehen.

Im US-Patent 4,942,036 wird ein Verfahren zur kontinuierlichen Dosierung von Wirkstoffen an den Körper oder das Skelett eines Warmblüters offenbart, wobei diese Dosierung über einen langen Zeitraum erfolgen soll. Zunächst werden bipolare Lipidvisikel hergestellt, die entsprechend zielgerichtet ausgestattet sind und die die Wirkstoffe enthalten können. Die Lipidvisikel zeigen eine gewisse Affinität zum Hydroxylapatit des Knochens und werden innerlich verabreicht.

Ein limitierender Faktor der Liposomen als Wirkstoffträger stellt ihre Zersetzung durch Makrophagen (Kupfer-Zellen) in der Leber und der Milz direkt nach intravenöser Gabe dar. Die Geschwindigkeit und der Umfang ihrer Aufnahme ist abhängig von der Membran-Rigidität, der Liposomen- Größe und der Dosis. Eine Modifikation der Liposomenoberfläche kann den unerwünschten Abbau durch Makrophagen vermindern. Durch das Anbinden von PEG-Einheiten an die äußere Membran kann die Zirkulationszeit deutlich erhöht werden (langzirkulierende Liposomen). Alternativ können auch zielgerichtete Moleküle (homing molecules) an die Liposomdoppelschichten angeheftet werden, um diese Strukturen spezifisch für den Wirkort zu machen, z.B. Immunoliposomen (Liposomen, die an ihrer Oberfläche kovalentgebundene Antikörper als zielgerichteten Liganden aufweisen), diese können auch mit langzirkulierenden Eigenschaft ausgestattet sein.

### Passive zielgerichtete Applikation

Langzirkulierende Liposomen haben die Tendenz, in Geweben zu akkumulieren, welche ein durchlässiges Endothelium aufzeigen. Diese "passiven Eigenschaften der zielgerichteten Applikation" sind sehr nützlich für die zielgerichtete Applikation an Tumorgewebe, da die Anordnung der Blugefäße der meisten Tumore ausreichend durchlässig für Liposomen ist. Da außerdem das lymphatische Gewebe in Tumoren zumeist nicht voll entwickelt ist, neigen die extravasierten Liposomen dazu, im Zwischenraum der Tumorgewebe zu bleiben. Langzirkulierende Liposomen wurden häufig als Träger für Krebstherapeutika eingesetzt, wie z.B. Doxorubicin, Cisplatin, Vincristine und Camphotecin.

### Cholesterol

Cholesterol ist von der Struktur hergesehen ein wichtiger Bestandteil von Zellmembranen. Es beeinflusst die physikalischen Eigenschaften der Membran, insbesondere ihre Fluidität. Es wird sehr häufig in der Pharmazeutischen Industrie eingesetzt, insbesondere als Bestandteil von Liposomen. Cholesterol hat die Eigenschaft Membranen steifer zu machen. Der Zusatz von Cholesterol überführt die Membran in einen geordneten, fluiden Zustand über einen großen Temperaturbereich. Außerdem wurde der Gebrauch neusynthetisierter Cholesterolderivate schon sehr früh untersucht.

Die zuvor beschriebenen Komponenten zeigen eine Vielzahl von positive Eigenschaften bei der Behandlung der genannten Erkrankungen sowie bei der Applikation von Wirkstoffen.

Gegenstand der vorliegenden Erfindung sind Bisphosphonsäuren und Derivate davon mit der folgenden Formel (I) 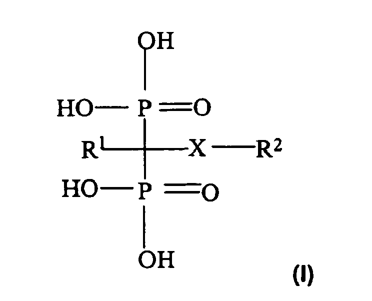 worin R¹ ist OH,
X ist:
(CH₃)ₘ-(OCR³HCH₂)ₙ-(O)ₒ-, in der R³ H oder CH₃ bedeutet und m für 0 oder eine Zahl von 1 bis 6, n für eine Zahl von 1 bis 10, insbesondere 1 bis 6 und o für 0 oder 1 steht,
-(CR⁴HCH₂O)ₚ- , R⁴ H oder CH₃ bedeutet, p für eine Zahl von 1 bis 10, insbesondere 1 bis 6, steht, oder (CH₃)_{q}-(OCR⁵HCH²)ᵣ-(O)ₛ-(CH₃)ₜ-, in der R⁵ H oder CH₃ bedeutet, und q für 0 oder eine Zahl von 1 bis 6, r für eine Zahl von 1 bis 10, insbesondere 1 bis 6
und s für 0 oder 1 und t für eine Zahl von 1 bis 6 steht,
R² ist ein Rest mit der Formel (II) 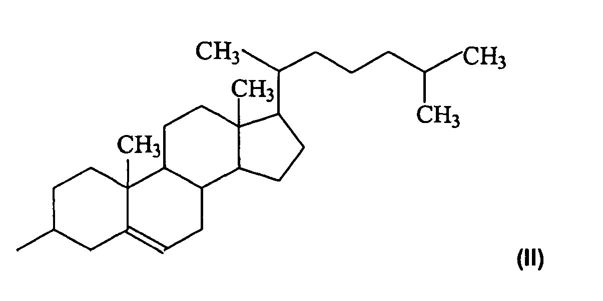 sowie deren physiologisch annehmbaren Salze und Trimethylsilylderivate.

Die erfindungsgemäßen Verbindungen eignen sich insbesondere zur Herstellung liposomaler Zubereitungen und zur Herstellung von Medikamenten, die zur Behandlung von Tieren und Menschen verwendet werden können, eingesetzt werden.

Die erfindungsgemäßen Bisphosphonsäureverbindungen können in Form ihrer Säuren aber auch als Salze oder Trimethylsilyl-Derivate vorliegen. In den Trimethylsilylderivaten ist wenigsten eine der OH-Gruppen am P durch eine Trimethylsilylgruppe ersetzt. Als Salze komme alle physiologisch verträglichen Salz in Betracht, insbesondere die Alkali-, Erdalkali- und Ammoniumsalze.

Besonders bevorzugt sind solche Verbindungen mit der Formel (I) in denen R¹ OH und R² ein Rest mit der allgemeinen Formel (II) (d.h. Cholesteryl-3-hydroxy-bisphosphonsäure) ist, ihre löslichen Salze davon, mit oder ohne Spacer-Molekül.

Die erfindungsgemäßen Verbindungen zeichnen sich durch eine Vielzahl von Anwendungsmöglichkeiten aus, wie z. B. als Chelat-Komplexbildner für di- und trivalente

Metallionen in technischen und industriellen Anwendungen, als Korrosionsschutzmittel in technischen und industriellen Anwendungen, als pharmazeutischer Wirkstoff, als Hilfsmittel für den Wirkstofftransport oder als Diagnostikum.

Die pharmazeutisch/pharmakologisch aktiven Substanzen können aus beliebigen Wirkstoffen ausgewählt sein, wie Krebstherpeutika, Virustatika, Antibiotika, antimykotische, antiinflammatorische, das Knochengewebe stimulierende oder das Knochengewebe unterdrückende Substanzen, wobei diese Aufzählung nicht abschließend ist. Als Antibiotika können insbesondere Aminoglykoside, Penicilline, Cephalosporine, Tetracycline, Makrolide, Lincosamide, Fluoroquinolone, Streptogramine, Nitroimidazole, Azole, Polyene, Polypeptid-Antibiotika, antibiotische Oligonukleotide, insbesondere Gentamycin, Amkacin oder Tobramycin, Nafcillin oder Piperacillin, Cefepim oder Cefuroxim, Tetracyclin oder Doxycyclin, Erythromycin, Clarithromycin oder Azithromycin, Klindamycin, Ciprofloxacin oder Moxifloxacin, Dalfopristin oder Quinupristin, Metronidazol, Miconazol oder Ketoconazol, Amphotericin B, Vancomycin oder Bacitracin genannt werden. Als Beispiele für Krebstherapeutika können Folsäureantagonisten, Alkylantien, Antimetabolite, Purinantagonisten, Pyrimidinantagonisten, Pflanzenalkaloide, Anthracycline, Hormonantagonisten, Aromataseinhibitoren, Bisphosphonate oder Antisense Oligonukleotide genannt werden.

Weitere Gruppe pharmazeutisch wirksame Substanzen können ausgewählt werden aus Sulfamethoxazol oder Sulfadiazin, Cisplatin oder Procarbazin, Methotrexat, Mercaptopurin, Fluorouracil oder Cytarabin, Vinblastin, Vincristin, Etoposid oder Paclitaxel, Doxorubicin, Epirubicin, Pirarubicin, oder Daunorubicin, Goserelin oder Aminoglutethimid, Etidronat, Pamidronat, Risedronat oder Clodronat.

In einer weiteren Ausführungsform werden die erfindungsgemäßen Bisphosphonsäuren in Gegenwart von sogenannten Duplexmolekülen eingesetzt, beispielsweise solchen, die aus kovalentgebundenen Fluoruracilen und Cytosinarabinosiden bestehen.

Die erfindungsgemäßen Verbindungen zeichnen sich durch eine Affinität zum Knochen auf und eignen sich daher sowohl als Hilfsmittel für den Wirkstofftransport als auch für den Transport von Diagnostika, in diesen Ausführungsformen sind die erfindungsgemäßen Verbindungen an ein aktives Agens (Wirkstoff) und/oder an ein Diagnostikum gebunden oder werden als Trägermaterialien für diese Substanzen eingesetzt. Beispiele für therapeutische Wirkstoffe sind z.B. Knochenkrebstherapeutika, die im Knochenwebe und im Knochenmark von Mensch und Tier eingesetzt werden.

In einer weiteren Ausführungsform können die erfindungsgemäßen Verbindungen und ihre Derivate als Transportmoleküle für divalente Kationen, insbesondere als zielgerichtete Liganden von Calciumionen für die Behandlung von Calcium-Metabolismus-Erkrankungen, eingesetzt werden.

Eine weitere Ausführungsform der vorliegenden Erfindung betrifft die Verwendung der erfindungsgemäßen Bisphosphonsäueren und ihrer Derivate zur Behandlung von Knochenmetastasen.

In der pharmazeutischen Anwendung werden die erfindungsgemäßen Verbindungen vorzugsweise in Kombination mit einem üblichen Trägermaterial und ggf. weiteren Hilfsmitteln eingesetzt.

Ein weiterer Gegenstand der vorliegenden Erfindung betrifft ein Verfahren zur Herstellung der Verbindungen mit der Formel I, in welchem eine Verbindung mit der Formel III, R²-X-COOH oder ein reaktives Derivat davon in an sich bekannter Weise mit der Bisphosphonsäure oder Tris(trimethylsilyl)phosphit umgesetzt und das erhaltene Produkte direkt isoliert oder durch Hydrolyse in die frei Phosphonsäure überführt wird. Die weitere Umsetzung in die physiologisch verträglichen Salze kann Umsetzung mit geeigneten Basen erfolgen.

In einer weiteren Ausgestaltung der vorliegenden Erfindung werden die erfindungsgemäßen Verbindungen mit der Formel I in einer Zusammensetzung in Gegenwart von geeigneten Konjugaten eingesetzt. Die Konjugate können ausgewählt sind aus Liposomen, Nanopartikeln, Nanospheres, Nanokapseln, Mizellen oder Polymersystemen. Vorzugsweise werden die Verbindungen der allgemeinen Formel (I) oder ihre Derivate in Kombination mit einer Mischung von Phospholipiden, einschließlich einem Uronsäure-Derivat als Ummantelungsmaterial oder einem anderen Ummantelungsmaterial, verwendet, wobei Menge, Art und Konzentration der einzelnen Bestandteile beliebig und in Abhängigkeit vom Anwendungszweck ausgewählt werden. Als Uronsäurederivate können z. B. Palmityl-D-glucuronid oder Galactosyl-D-glucuronid in Konzentrationen von 0,1 bis 25 mol% eingesetzt werden. In dieser Ausgestaltung ist das erfindungsgemäße Bisphoshphonsäurederivat an ein langzirkulierendes Liposom gebunden, welches mit einem Uron-Säure-Derivat als Ummantelungssubstanz modifiziert ist.

Als Phospholipide kann die Zusammensetzung Phosphatidylcholin, Phosphatidylglycerol, Phosphatidylethanolamin, Phosphatidylinositol, Phosphatidylsäure, Sphingomyelin, Ceramid in ihren natürlichen, halbsynthetischen oder synthetischen Formen sowie Stearylamin und Cholesterol umfassen, wobei ein Phospholipidgemisch, das Dipalmitoylphosphatidylcholin und Dimyristoylphosphatidylglycerol enthält, besonders bevorzugt ist. Als Polymere kann die Zusammensetzung Polyvinylpyrrolidone oder Polyethylenoxide enthalten.

Diese Zusammensetzung kann auch eine oder mehrere der oben beschriebenen Aktivsubstanzen in einer beliebigen Konzentration enthalten. Diese Substanzen können ausgewählt sein aus den bereits beschriebenen pharmazeutisch wirksamen Substanzen und Diagnostika, aber auch aus Desinfektionsmitteln, Chemikalien und Magnetpartikeln.

Ein weiterer Gegenstand der vorliegenden Erfindung betrifft ein Verfahren zur Herstellung eienr liposomalen Zubereitung, worin eine Rohmischung der einzelnen Bestandteile, wie Palmityl-D-glucuronid, Phospholipiden, Bisphosphonsäure(n) oder ein Derivat davon mit der allgemeinen Formel (I) und beliebige einzelne oder Kombinationen von aktiven Substanzen durch Ultraschall, Hochdruckextrusion, oder Hochdruckhomogenisation miteinander vermischt werden. Es wird ein Liposomenprodukt erhalten, das vorzugsweise einen mittleren Partikeldurchmesser von 30 bis 1000 nm aufweist.

Die erfindungsgemäße liposomale Zusammensetzung wird vorzugsweise in eine wässrige Dispersion oder ein Lyophilisat. Diese Zubereitungen sind insbesondere zur Herstellung von pharmazeutischen Formulierungen, wie zur Injektion oder Inhalation, geeignet. Diese Formulierungen enthalten üblicherweise einen oder mehrere Aktivsubstanzen. Als Beispiele können die bereits genannten Krebstherapeutika, Antibiotika oder Antisense Oligonukleotide angeführt werden.

Ein weiterer Gegenstand der vorliegenden Anmeldung betrifft die Verwendung der erfindungsgemäßen Verbindungen mit der Formel I zur Herstellung eines Medikaments zur Behandlung von Human- und Tierkrankheiten. Die Applikation dieser Mittel kann auf intravenöse oder orale Weise oder jede beliebige andere Weise erfolgen.

### Beispiele

### Vergleichsbeispiel 1: Zubereitung von Cholesteryl-3-hydroxy-bisphosphonsäure

Cholesterylchlorid wurde über die entsprechende Grignardverbindung in die Carbonsäure überführt (Ausbeute: 35%). Dieses Produkt wird dann in Gegenwart von Oxalylchlorid in das Säurechlorid umgewandelt (Ausbeute: 95%).

6,5 g (0,015 mol) Säurechlorid wird in 150 mL THF gelöst. Unter Stickstoff wird langsam 13.4 g (0,045 mol) P(OSiMe3)₃ bei 0°C zugegeben. Das Gemisch wird bei Raumtemperatur 3 h gerührt.

Danach werden 0,5 mL (0,03 mol) Wasser zugefügt und die flüchtigen Bestandteile werden im Vakuum bei 90°C abgezogen.

Der Feststoff wird in Ethylacetat gelöst und 1 h unter Rückfluss gekocht.

Dann wird abfiltriert und der verbleibende Feststoff zweimal mit Hexan gewaschen. Das Produkt wurde im Vakuum (0,001 Torr) getrocknet (Ausbeute: 81%). Cholesteryl-3-hydroxy-bisphosphonsäure: MS: Molekülion *m*/*z* 561 [M+H]⁺; ³¹p-NMR 6 = 21.6 ppm.

### Vergleichsbeispiel 2: Leerliposomen bestehend aus Dodecan-bisphosphonsäure

Liposomen enthaltend Soja-Phosphatidylcholin, Cholesterol, Palmityl-D-glucuronid und Dodecanbisphosphonsäure in einem molaren Verhältnis von 1,0:0,3:0,1:0,1 (100 mg/ml) wurden mit Ultraschall hergestellt. Der Teilchendurchmesser betrug 120 ± 40 nm. Er wurde durch Photonenkorrelationsspektroskopie (light scattering) bestimmt.

### Vergleichsbeispiel 3: Leerliposomen bestehend aus Palmitylbisphosphonsäure

Liposomen enthaltend Soja-Phosphatidylcholin, Phosphatidylglycerol, Palmityl-D-glucuronid und Palmitylbisphosphonsäure in einem molaren Verhältnis von 1,0:0,2:0,1:0,1 (100 mg/ml) wurden mit Ultraschall hergestellt. Der Teilchendurchmesser betrug 120 ± 40 nm. Er wurde durch Photonenkorrelationsspektroskopie (light scattering) ermittelt.

### Vergleichsbeispiel 4: Leerliposomen bestehend aus Cholesteryl-3-hydroxy-bisphosphonsäure

Liposomen enthaltend Soja-Phosphatidylcholin, Cholesterol, Palmityl-D-glucuronid und Cholesteryl-3-hydroxy-bisphosphonsäure in einem molaren Verhältnis von 0,5:0,14:0,05:0,03 (50 mg/ml) wurden mit Ultraschall und Hochdruckfiltration hergestellt. Der Teilchendurchmesser betrug 120 ± 40 nm. Er wurde durch Photonenkorrelationsspektroskopie (light scattering) ermittelt.

### Beispiel 5: Cholesterylbisphosphonsäuren mit Oxyethylen-Bausteinen als "Spacer" zwischen Steroid und Säurefunktion.

Alle verwendeten Lösungsmittel wurden sorgfältig getrocknet, die Diole (Ethylenglykol, Triethylenglykol) über Calciumhydrid destilliert und die Reaktionen in einer Atmosphäre von trockenem Stickstoff durchgeführt.

### 1. Cholesteryl-toluol-p-sulfonat

Zu einer Lösung von 30 g (77.6 mmol) Cholesterin in 250 ml Pyridin, wurden 22.2 g (116.4 mmol) Toluol-*p*-sulfonylchlorid gegeben. Die Reaktionsmischung wurde für 24 h bei Raumtemperatur gerührt und anschließend 200 ml Eiswasser langsam dazugegeben. Der gelbliche Niederschlag wurde filtriert, mit Ethanol gewaschen (3×70 ml). Das Produkt war ein weißes Pulver in 95% Ausbeute (39.9 g).

### 2. Cholesteryl-hydroxyethylether

Eine Lösung von 1 mmol Cholesteryl-toluene-p-sulfonate und 200 mmol von Ethylen- oder Triethylenglykol in Dioxan wurde während 2.5 h zum Sieden erhitzt. Nach dem Abpumpen des Lösungsmittels im Vakuum wurde das verbleibende Öl in Diethylether aufgenommen und mit Wasser gewaschen. Die organische Phase wurde mit Magnesiumsulfat getrocknet, dann abfiltriert und das Lösungsmittel abgezogen. Im Falle von **Cholesteryl-hydroxyethylether** wurde der feste Rückstand mit Hexan gewaschen. Es verblieb ein weißes Pulver in 86% Ausbeute. Im Falle von **Cholesteryl-3,6-dioxaoctan-1-ol** war eine Reinigung über Silicagel notwendig. Gelbe Verunreinigungen wurden mit Hexan ausgewaschen. Das Produkt wurde mit einem (90/10) Dichloromethan-Methanol-Gemsich eluiert und die Lösungsmittel im Vakuum abgepumpt. Der Rückstand war das gewünschte Produkt in 74% Ausbeute.

### 3. Cholesteryloxyethyl-Carbonsäuren

1.1 mmol *n*BuLi (1.4 M Lösung in Hexan) wurde zu einer Lösung von **Cholesterin** sowie der **Hydroxyethylether** (siehe 2) in THF bei -78°C gegeben, die Reaktionsmischung 10 min. gerührt und mit einem zweifachen Überschuß von Lithiumbromacetat versetzt. Nach 20 min. wurde auf Raumtemperatur erwärmt. Nach 16 h bei Raumtemperatur wurde die Mischung für 3 h auf 60°C gehalten. Das Lösungsmittel wurde im Vakuum entfernt, Diethylether wurde dazugegeben und die organische Phase mehrere Male mit Wasser gewaschen, und im Vakuum getrocknet. **2-(Cholesteryloxy)essigsäure⁵** wurde in 85% Ausbeute isoliert, **[2-(Cholesteryloxy)ethoxy]essigsäure** in 60% Ausbeute nach Umkristallisation aus Hexan: **{2-[(Cholesteryl-3,6-dioxaoctan-1-ol)oxy]ethoxy}essigsäure** wurde in 70% Ausbeute isoliert, indem eine Lösung des Rohprodukts in einer (1/1) Ether/Pentan-Gemisch durch eine Säule mit Silicagel geschickt und dann mit einem (5/1) Dichloromethan/Methanol-Gemisch eluiert wurde.

### 4. Synthese der entsprechenden Bisphosphonsäuren

Die Carbonsäuren aus 3 wurden in Dichloromethan mittels Oxalylchlorid in die Säurechloride überführt, die dann ohne weitere Reinigung mit Tris(trimethylsilyl)phosphit in Ether umgesetzt wurden, gefolgt von der sauren Hydrolyse der entstehenden trimethylsilylierten Bisphosphonsäureester, die zu den gewünschten freien Bisphosphonsäuren in 80-95% Ausbeute führte. 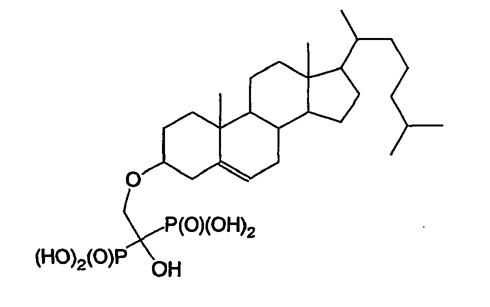 aus 2-(Cholesteryloxy)essigsäure

*2-(2,3,4,7,8,9,10,11,12,13,14, 15,16,17-tetradecahydro-10,13-dimethyl-17-(6-methylheptan-2-yl)-1H-cyclopenta[a]phenanthren-3-yloxy)-1-hydroxy-1-phosphono)ethylphosphonsäure;* C₂₉H₅₂O₈P₂ (590.31); NMR: ¹H (DMSO-d₆): 0.3-2.7 ppm (m, 43 H), 3.3 ppm (1 H), 4.2 ppm (m, 2 H), 5.4 ppm (breit, 1 H), 10.2 ppm (breit, 5H); ³¹P (DMSO-d₆):18.8 ppm (t, J =11.99 Hz). 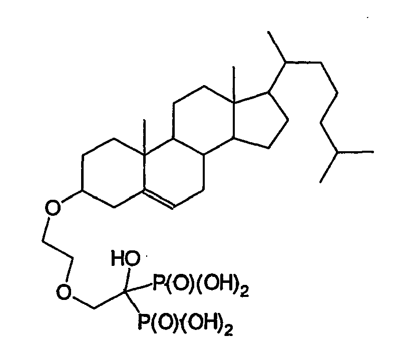 aus [12-(Cholesteryloxy)ethoxy]essigsäure

*2-(2-(2,3,4,7,8,9,10,11,12,13,14,15,16,17-tetradecahydro-10,13-dimethyl-17-(6-methylheptan-2-yl)-1H-cyclopenta[a]phenanthren-3-yloxy)ethoxy)-1-hydroxy-1-phosphono)ethylphosphonsäure;* C₃₁H₅₆O₉P₂ (634.3); NMR: ¹H (DMSO-d₆): 0.59-2.53 ppm (m, 43 H), 3.28 ppm (m, 1 H), 3.72 ppm (m, 4 H), 4.17 ppm (m, 2 H), 5.37 ppm (m, 1 H), 10.4 ppm (breit, 5 H); ³¹P (DMSO-d₆): 20.1 ppm (t, J = 13.87 Hz). 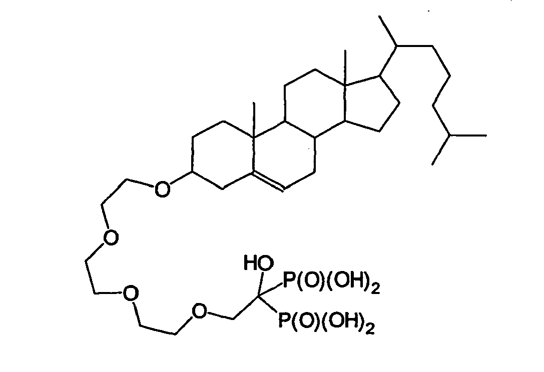 aus {2-[(Cholesteryl-3,6-dioxaoctan-1-ol)oxy]ethoxy}essigsäure

*2-(2-(2-(2-(2,3,4,7,8,9,10,11,12,13,14,15,16,17-tetradecahydro-10,13-dimethyl-17-(6-methylheptan-2yl)-1H-cydopenta[a]phenanthren-3-yloxy)ethoxy)ethoxy)ethoxy)-1-hydroxy-1-phosphono)ethylphosphonsäure;* C₃₅H₆₄O₁₁P₂ (722.39); NMR: ¹H (DMSO-d₆): 0.20-2.39 ppm (m, 43 H), 3.3 ppm (m, 1 H), 3.49 ppm (m, 12H), 5.28 ppm (breit, 1 H), 3.82 ppm (t, 2 H, J = 10.99 Hz), 10.8 ppm (breit, 5 H); ³¹P (DMSO-d₆) 18.8 ppm (t, J = 10.9 Hz).

## Patentansprüche

1. Bisphosphonsäure mit der allgemeinen Formel (I) 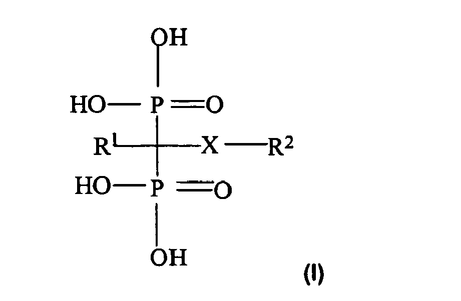 worin R¹ ist OH,
X ist:
(CH₂)ₘ-(OCR³HCH₂)ₙ-(O)ₒ-, in der R³ H oder CH₃ bedeutet und m für 0 oder eine Zahl von 1 bis 6, n eine Zahl von 1 bis 10, insbesondere 1 bis 6 und o für 0 oder 1 steht,
-(CR⁴HCH₂O)ₚ-, R⁴ H oder CH₃ bedeutet, p für eine Zahl von 1 bis 10, insbesondere 1 bis 6, steht, oder
(CH₂)_{q}-(OCR⁵HCH₂)ᵣ-(O)ₛ-(CH₂)ₜ-, in der R⁵ H oder CH₃ bedeutet, und q für 0 oder eine Zahl von 1 bis 6, r für eine Zahl von 1 bis 10, insbesondere 1 bis 6 und s für 0 oder 1 und t für eine Zahl von 1 bis 6 steht,
R² ein Rest mit der Formel (II) ist 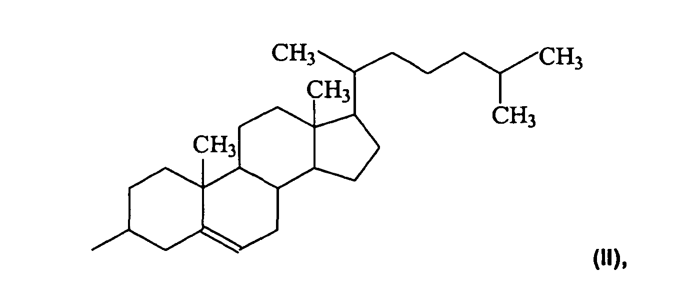 sowie deren physiologisch annehmbaren Salze und Trimethylsilylderivate.

2. Verwendung der Bisphosphonsäuren nach Anspruch 1 als Chelat-Komplexbildner oder Transportmittel für di- und trivalente Metallionen in technischen und industriellen Anwendungen, als Korrosionsschutzmittel in technischen und industriellen Anwendungen.

3. Verwendung der Bisphosphonsäuren nach Anspruch 1 zur Herstellung eines Medikaments.

4. Verwendung der Bisphosphonsäuren nach Anspruch 3 als Hilfsmittel für den Wirkstofftransport oder als Diagnostikum.

5. Verwendung nach Anspruch 4, **dadurch gekennzeichnet, dass** die Verbindung mit der allgemeinen Formel (I) an ein aktives Agens oder an ein Diagnostikum gebunden ist.

6. Verwendung nach einem der Ansprüche 2 bis 5, **dadurch gekennzeichnet, dass** das aktive Agens oder das Diagnostikum ausgewählt ist aus Krebstherpeutika, Virustatika, Antibiotika, antimykotische, antiinflammatorische, das Knochengewebe stimulierende oder das Knochengewebe unterdrückende Substanzen.

7. Verwendung nach einem der Ansprüche 2 bis 6 in Kombination mit oder als Bestandteil von Liposomen, Nanopartikeln, Nanospheres, Nanokapseln, Mizellen oder Polymersystemen.

8. Verfahren zur Herstellung der Verbindungen mit der Formel I, in welchem eine Verbindung mit der Formel III, R²-X-COOH oder ein reaktives Derivat davon in an sich bekannter Weise mit Tris(trimethylsilyl)phosphit umgesetzt und das erhaltene Produkte direkt isoliert oder durch Hydrolyse in die frei Phosphonsäure überführt wird.

9. Liposomale Zusammensetzung enthaltend eine Verbindung mit der allgemeinen Formel I, Phospholipide und/oder einem Uronsäure-Derivat.

10. Liposomale Zusammensetzung nach Anspruch 9, **dadurch gekennzeichnet, dass** das als Uronsäurederivate Palmityl-D-glucuronid und/oder Galactosyl-D-glucuronid in Konzentrationen von 0,1 bis 25 mol% enthalten sind.

11. Liposomale Zusammensetzung nach Anspruch 9 oder 10, **dadurch gekennzeichnet, dass** die Phospholipie ausgewählt sind aus Phosphatidylcholin, Phosphatidylglycerol, Phosphatidylethanolamin, Phosphatidylinositol, Phosphatidylsäure, Sphingomyelin, Ceramid in ihren natürlichen, halbsynthetischen oder synthetischen Formen sowie Stearylamin und Cholesterol.

12. Liposomale Zusammensetzung nach einem der Ansprüche 9 bis 11, durch gekennzeichnet, dass sie als wässerige Dispersion oder als Lyophilisat vorliegt.

13. Verfahren zur Herstellung einer liposomalen Zubereitung nach den Ansprüchen 9 bis 12, worin eine Rohmischung der einzelnen Bestandteile, wie Palmityl-D-glucuronid, Phospholipiden, Bisphosphonsäure(n) oder ein Derivat davon mit der allgemeinen Formel (I) und beliebige einzelne oder Kombinationen von aktiven Substanzen durch Ultraschall, Hochdruckextrusion, oder Hochdruckhomogenisation miteinander vermischt werden.

14. Verwendung einer liposomalen Zusammensetzung nach einem der Ansprüche 9 bis 12 zur Herstellung eines Medikaments zur Behandlung von Human- und Tierkrankheiten.

## Claims

1. Biphosphonic acid of the general formula (I) 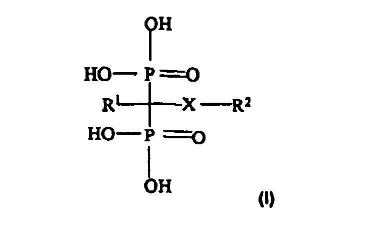 wherein R¹ is OH,
X is:
(CH₂)ₘ-(OCR³HCH₂)ₙ-(O)ₒ-, wherein R³ is H or CH₃ and m denotes 0 or a number from 1 to 6, n denotes a number of 1 to 10, in particular 1 to 6 and o denotes 0 or 1,
-(CR⁴HCH₂O)ₚ-, wherein R⁴ is H or CH₃, p denotes a number from 1 to 10, in particular 1 to 6, or
(CH₂)_{q}-(OCR⁵HCH₂)ᵣ-(O)ₛ-(CH₂)ₜ-, wherein R⁵ is H or CH₃ and q denotes 0 or a number from 1 to 6,
r denotes a number from 1 to 10, in particular 1 to 6, and s denotes 0 or 1 and t denotes a number of 1 to 6,
R² is a radical of formula (II) 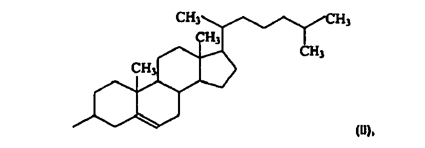 as well as the physiologically acceptable salts and trimethylsilyl derivates thereof.

2. Use of the biphosphonic acids as claimed in Claim 1 as chelating agents or as carriers for di- and trivalent metal ions in technical and industrial applications, as corrosion inhibitors in technical and industrial applications.

3. Use of the biphosphonic acids as claimed in Claim 1 for producing a medicament.

4. Use of the biphosphonic acids as claimed in Claim 3 as an expedient for transporting an active ingredient or as a diagnostic agent.

5. Use as claimed in Claim 4, **characterised in that** the compound of general formula (I) is bound to an active agent or to a diagnostic agent.

6. Use as claimed in any one of Claims 2 to 5, **characterised in that** the active agent or the diagnostic agent is selected from anti-cancer drugs, antiviral agents, antibiotics, anti-fungal, anti-inflammatory substances or bone tissue stimulating substances or bone tissue suppressing substances.

7. Use as claimed in any one of Claims 2 to 6 in combination with or as a component of liposomes, nanoparticles, nanospheres, nanocapsules, micelles or polymer systems.

8. A method for producing the compounds of formula I, wherein a compound of formula III, R²-X-COOH or a reactive derivate thereof is reacted in a manner known per se with tris(trimethylsilyl) phosphite and the product obtained is directly isolated or transferred into free phosphonic acid by way of hydrolysis.

9. A liposomal composition containing a compound of general formula I, phospholipids and/or a uronic acid derivative.

10. The liposomal composition as claimed in Claim 9, **characterised in that** as uronic acid derivates, palmityl-D-glucuronide and/or galactosyl-D-glucuronide are contained in concentrations of 0.1 to 25 mol%.

11. The liposomal composition as claimed in Claim 9 or 10, **characterised in that** the phospholipids are selected from phosphatidylcholine, phosphatidylglycerol, phosphatidylethanolamine, phosphatidylinositol, phosphatidic acid, sphingmyelin, ceramide in their natural, semisynthetic or synthetic forms as well as stearylamine and cholesterol.

12. The liposomal composition as claimed in any one of Claims 9 to 11, **characterised in that** it is present as an aqueous disperson or as a lyophilisate.

13. A method for producing a liposomal preparation as claimed in any one of Claims 9 to 12, wherein a raw mixture of the individual components such as palmityl-D-glucuronide, phospholipids, biphosphonic acid(s) or a derivate thereof of the general formula (I) and any desired active substances or combinations thereof is mixed together by means of ultrasound, high-pressure extrusion or high-pressure homogenisation.

14. The use of a liposomal composition as claimed in any one of Claims 9 to 12 for producing a medicament for treating human and animal diseases.

## Revendications

1. Acide bisphosphonique de la formule générale (I) 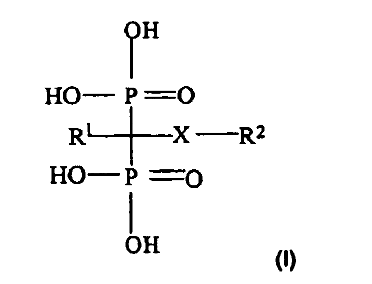 dans laquelle R¹ est un OH
X est:
(CH₂)ₘ-(OCR³HCH₂)ₙ-(O)ₒ-, dans lequel R³ signifie un H ou un CH₃ et m représente 0 ou un nombre de 1 à 6, n un nombre de 1 à 10, en particulier de 1 à 6 et o représente 0 ou 1,
-(CR⁴HCH₂O)ₚ-, R⁴ signifiant H ou CH₃, p représentant un nombre de 1 à 10, en particulier de 1 à 6, ou
(CH₂)_{q}-(OCR⁵HCH₂)ᵣ-(O)ₛ-(CH₂)ₜ-, dans lequel le R⁵ signifie H ou CH₃, et
q représente 0 ou un nombre de 1 à 6, r un nombre de 1 à 10, en particulier de 1 à 6 et s représente 0 ou 1 et t représente un nombre de 1 à 6,
R² est un radical de la formule (II) 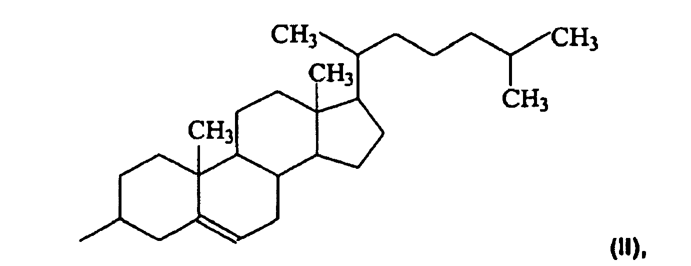 ainsi que ses sels et ses dérivés triméthylsilyle physiologiquement acceptables.

2. Utilisation des acides bisphosphoniques selon la revendication 1 comme chélatants ou comme agents de transport pour des ions métalliques di- et trivalents dans des applications techniques et industrielles, comme anticorrosion dans des applications techniques et industrielles.

3. Utilisation des acides bisphosphoniques selon la revendication 1 à la préparation d'un médicament.

4. Utilisation des acides bisphosphoniques selon la revendication 3 comme auxiliaires pour le transport des principes actifs ou comme agents de diagnostic.

5. Utilisation selon la revendication 4, **caractérisée en ce que** le composé de la formule générale (I) est lié à un agent actif ou à un agent de diagnostic.

6. Utilisation selon l'une quelconque des revendications 2 à 5, **caractérisée en ce que** l'agent actif ou l'agent de diagnostic est choisi parmi les produits de thérapie du cancer, les virostatiques, les antibiotiques, les substances antimycotiques, anti-inflammatoires, stimulatrices des tissus osseux ou inhibitrices des tissus osseux.

7. Utilisation selon l'une quelconque des revendications 2 à 6 en combinaison avec ou comme constituant de liposomes, de nanoparticules, de nanosphères, de nanocapsules, de micelles ou de systèmes de polymères.

8. Procédé de préparation des composés de la formule I, dans lequel un composé de la formule III, R²-X-COOH ou un dérivé réactif de ceux-ci est mis en réaction de la façon bien connue avec du tris(triméthylsilyl)phosphite et dans lequel le produit obtenu est isolé directement ou est transformé par hydrolyse en l'acide phosphonique libre.

9. Composition liposomale contenant un composé de la formule générale I, des phospholipides et/ou un dérivé d'acide uronique.

10. Composition liposomale selon la revendication 9, **caractérisée en ce que** comme dérivé d'acide uronique, le palmityl-D-glucuronide et/ou le galactosyl-D-glucuronide sont contenus en des concentrations de 0,1 à 25 % en mole.

11. Composition liposomale selon la revendication 9 ou 10, **caractérisée en ce que** les phospholipides sont choisis parmi la phosphatidylcholine, le phosphatidylglycérol, la phosphatidyléthanolamine, le phosphatidylinositol, l'aide phosphatidique, la sphingomyéline, le céramide dans leurs formes naturelles, semi-synthétiques ou synthétiques, ainsi que la stéarylamine et le cholestérol.

12. Composition liposomale selon l'une quelconque des revendications 9 à 11, **caractérisée en ce qu'**elle se présente comme dispersion aqueuse ou comme produit lyophilisé.

13. Procédé de production d'une préparation liposomale selon l'une quelconque des revendications 9 à 12, dans lequel un mélange brut des différents composants comme le palmityl-D-glucuronide, des phospholipides, un ou plusieurs acides bisphosphoniques ou un dérivé de ceux-ci avec la formule générale (I) et des substances actives quelconques individuelles ou en combinaison, sont mélangés ensemble par ultrasons, par extrusion sous haute pression ou par homogénéisation sous haute pression.

14. Utilisation d'une composition liposomale selon l'une quelconque des revendications 9 à 12 à la préparation d'un médicament pour le traitement de maladies humaines et animales.
